# EUROPEAN PATENT SPECIFICATION

(11) **EP 1 339 407 B1**
(45) Date of publication and mention of the grant of the patent: **19.04.2006**
(21) Application number: 01998349.3
(22) Date of filing: 20.11.2001
(51) Int. Cl.: A61K 31/47, A61P 1/00

(54) **FARNESYL PROTEIN TRANSFERASE INHIBITORS FOR THE TREATMENT OF INFLAMMATORY BOWEL DISEASE**
FARNESYL-PROTEIN-TRANSFERASEHEMMER ZUR BEHANDLUNG DER ENTZÜNDLICHEN DARMERKRANKUNG
INHIBITEURS DE FARNESYL PROTEINE TRANSFERASE DESTINES AU TRAITEMENT D'UNE MALADIE INTESTINALE INFLAMMATOIRE

(30) Priority: 28.11.2000 US 253346 P
(43) Date of publication of application: 03.09.2003
(73) Proprietor: JANSSEN PHARMACEUTICA N.V., 2340 Beerse (BE)
(72) Inventor: END, David, William, Ambler, PA 19002 (US); BOWDEN, Charles, Ronald, North Wales, PA 19454 (US)
(86) International application number: PCT/EP2001/013540
(87) International publication number: WO 2002/043733

(56) References cited:
- EP-A- 0 664 128
- WO-A-00/39082
- WO-A-98/20001
- WO-A-98/40383
- WO-A-98/43629
- WO-A-98/49157
- WO-A-99/01434
- WO-A-99/26657
- GB-A- 2 294 462
- US-A- 5 470 832
- US-A- 5 929 077
- US-A- 5 968 952
- US-A- 6 037 350

## Description

The present invention relates to the new use of a specific famesyl protein transferase inhibitor in the manufacture of a medicament for the treatment of inflammatory bowel disease.

Inflammatory bowel disease (IBD) identifies a group of chronic inflammatory disorders involving the (mainly) lower gastrointestinal tract. Although the exact aetiology of these disorders remains elusive, enviromental, genetic and immunological factors are implicated. Inflammatory bowel diseases such as Crohn's disease and ulcerative colitis are chronic progressive inflammatory conditions, with high morbidity, and potentially life-threatening complications (e.g. toxic megacolon, increased risk for colon cancer).

Ulcerative colitis involves an inflammatory reaction affecting the colon. The colon appears ulcerated and hemorrhaged. The inflammation is generally uniform and continuous and often involves the rectum. The major symptoms of ulcerative colitis include bloody diarrhea or constipation, abdominal pain, signs of dehydration, anemia, fever and weight loss. Extracolonic manifestations may include arthritis, evidence of liver disease, skin disease and lung disease.

Crohn's disease is characterized by inflammation extending through all layers of the intestional wall and often including the mesenteric lymph nodes. The inflammations may penetrate the mucosa and coalesce to form channels called fistulas and fissures. In Crohn's disease inflammations of the bowel are often discontinous and often granulomatous (see Horrison's Priciples of Internal Medicine, thirteenth ed., 1994, by McGraw-Hill, Inc. ISBN 0-07-032370-4, pg., 1403-1416).

Current therapies include but are not limited to treatment with aminosalicylates (e.g. sulfasalazine, olsalazine), corticosteroids (prednisolone, budenoside), and immuno-modulating drugs (azathioprine, 6-MP). Molecules which target specific steps in the inflammatory cascade, have been recently introduced or are under investigation for the treatment of IBD. However, approximately 25% of IBD patients will require surgery (colectomy) during the course of the disease. Surgical procedures are expensive, and associated with increased mortality and morbidity. There is undoubtedly a need for effective treatment of IBD. WO 98/43629 relates to the use of farnesyl protein transferase inhibitors in the treatment of inflammatory bowel disease.

It is, therefore, an object of the present invention to provide a new method for the treatment of inflammatory bowel disease.

WO-97/21701 describes the preparation, formulation and pharmaceutical properties of famesyl protein transferase inhibiting (imidazoly-5-yl)methyl-2-quinolinone derivatives of formulae (I), (II) and (III), as well as intermediates of formula (II) and (III) that are metabolized *in vivo* to the compounds of formula (I). The compounds of formulas (1), (II) and (III) are represented by 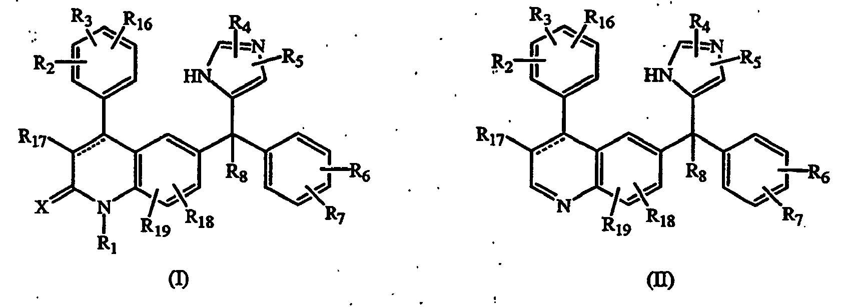 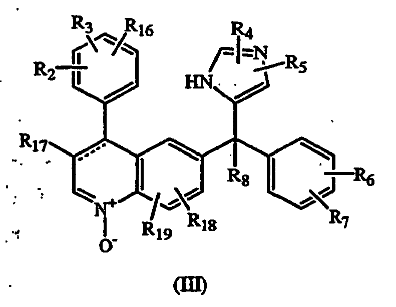 the pharmaceutically acceptable acid or base addition salts and the stereochemically isomeric forms thereof, wherein
the dotted line represents an optional bond;
X is oxygen or sulfur;
R¹ is hydrogen, C₁₋₁₂alkyl, Ar¹, Ar²C₁₋₆alkyl, quinolinylC₁₋₆alkyl, pyridylC₁₋₆alkyl, hydroxyC₁₋₆alkyl, C₁₋₆alkyloxyC₁₋₆alkyl, mono- or di(C₁₋₆alkyl)aminoC₁₋₆alkyl, aminoC₁₋₆alkyl, or a radical of formula -Alk¹-C(=O)-R⁹, -Alk¹-S(O)-R⁹ or -Alk¹-S(O)₂-R⁹, whereinAlk¹ is C₁₋₆alkanediyl,
R⁹ is hydroxy, C₁₋₆alkyl, C₁₋₆alkyloxy, amino, C₁₋₈alkylamino or C₁₋₈alkylamino substituted with C₁₋₆alkyloxycarbonyl;
R², R³ and R¹⁶ each independently are hydrogen, hydroxy, halo, cyano, C₁₋₆alkyl, C₁₋₆alkyloxy, hydroxyC₁₋₆alkyloxy, C₁₋₆alkyloxyC₁₋₆alkyloxy, aminoC₁₋₆alkyloxy, mono- or di(C₁₋₆alkyl)aminoC₁₋₆alkyloxy, Ar¹, Ar²C₁₋₆alkyl, Ar²oxy, Ar²C₁₋₆alkyloxy, hydroxycarbonyl, C₁₋₆alkyloxycarbonyl, trihalomethyl, trihalomethoxy, C₂₋₆alkenyl, 4,4-dimethyloxazolyl; or
when on adjacent positions R² and R³ taken together may form a bivalent radical of formula

-O-CH₂-O- (a-1),

-O-CH₂-CH₂-O- (a-2),

-O-CH=CH- (a-3),

-O-CH₂-CH₂- (a-4),

-O-CH₂-CH₂-CH₂- (a-5),

or

-CH=CH-CH=CH- (a-6);

R⁴ and R⁵ each independently are hydrogen, halo, Ar¹, C₁₋₆alkyl, hydroxyC₁₋₆alkyl, C₁₋₆alkyloxyC₁₋₆alkyl, C₁₋₆alkyloxy, C₁₋₆alkylthio, amino, hydroxycarbonyl, C₁₋₆alkyloxycarbonyl, C₁₋₆alkylS(O)C₁₋₆alkyl or C₁₋₆alkylS(O)₂C₁₋₆alkyl;
R⁶ and R⁷ each independently are hydrogen, halo, cyano, C₁₋₆alkyl, C₁₋₆alkyloxy, Ar²oxy, trihalomethyl, C₁₋₆alkylthio, di(C₁₋₆alkyl)amino, or when on adjacent positions R⁶ and R⁷ taken together may form a bivalent radical of formula

-O-CH₂-O- (c-1),

or

-CH=CH-CH=CH- (c-2);

R⁸ is hydrogen, C₁₋₆alkyl, cyano, hydroxycarbonyl, C₁₋₆alkyloxycarbonyl, C₁₋₆alkylcarbonylC₁₋₆alkyl, cyanoC₁₋₆alkyl, C₁₋₆alkyloxycarbonylC₁₋₆alkyl, carboxyC₁₋₆alkyl, hydroxyC₁₋₆alkyl, aminoC₁₋₆alkyl, mono- or di(C₁₋₆alkyl)aminoC₁₋₆alkyl, imidazolyl, haloC₁₋₆alkyl, C₁₋₆alkyloxyC₁₋₆alkyl, aminocarbonylC₁₋₆alkyl, or a radical of formula

-O-R¹⁰ (b-1),

-S-R¹⁰ (b-2),

-N-R¹¹R¹² (b-3),

wherein
= R¹⁰ is hydrogen, C₁₋₆alkyl, C₁₋₆alkylcarbonyl, Ar¹, Ar²C₁₋₆alkyl, C₁₋₆alkyloxycarbonylC₁₋₆alkyl, or a radical or formula -Alk²⁻OR¹³ or -Alk²-NR¹⁴R¹⁵;
R¹¹ is hydrogen, C₁₋₁₂alkyl, Ar¹ or Ar²C₁₋₆alkyl;
R12 is hydrogen, C₁₋₆alkyl, C₁₋₁₆alkylcarbonyl, C₁₋₆alkyloxycarbonyl, C₁₋₆alkylaminocarbonyl, Ar¹, Ar²C₁₋₆alkyl, C₁₋₆alkylcarbonylC₁₋₆alkyl, a natural amino acid, Ar¹ carbonyl, Ar²C₁-salkylcarbonyl, aminocarbonylcarbonyl, C₁₋₆alkyloxyC₁₋₆alkylcarbonyl, hydroxy, C₁₋₆alkyloxy, aminocarbonyl, di(C₁₋₆alkyl)aminoC₁₋₆alkylcarbonyl, amino, C₁₋₆alkylamino, C₁₋₆alkylcarbonylamino, or a radical or formula -Alk²-OR¹³ or -Alk²-NR¹⁴R¹⁵;
wherein
Alk² is C₁₋₆alkanediyl;
R¹³ is hydrogen, C₁₋₆alkyl, C₁₋₆alkylcarbonyl, hydroxy-C₁₋₆alkyl, Ar¹ or Ar²C₁₋₆alkyl;
R¹⁴ is hydrogen, C₁₋₆alkyl, Ar¹ or Ar²C₁₋₆alkyl;
R¹⁵ is hydrogen, C₁₋₆alkyl, C₁₋₆alkylcarbonyl, Ar¹ or Ar²C₁₋₆alkyl;
R¹⁷ is hydrogen, halo, cyano, C₁₋₆alkyl, C₁₋₆alkyloxycarbonyl, Ar¹;
R¹⁸ is hydrogen, C₁₋₆alkyl, C₁₋₆alkyloxy or halo;
R¹⁹ is hydrogen or C₁₋₆alkyl;
Ar¹ is phenyl or phenyl substituted with C₁₋₆alkyl, hydroxy, amino, C₁₋₆alkyloxy or halo; and
Ar² is phenyl or phenyl substituted with C₁₋₆alkyl, hydroxy, amino, C₁₋₆alkyloxy or halo.

As used in the foregoing definitions and hereinafter for compounds of formulae (I), (II), and (III) halo defines fluoro, chloro, bromo and iodo; C₁₋₆alkyl defines straight and branched chained saturated hydrocarbon radicals having from 1 to 6 carbon atoms such as, for example, methyl, ethyl, propyl, butyl, pentyl, hexyl and the like; C₁₋₈alkyl encompasses the straight and branched chained saturated hydrocarbon radicals as defined in C₁₋₆alkyl as well as the higher homologues thereof containing 7 or 8 carbon atoms such as, for example heptyl or octyl; C₁₋₁₂alkyl again encompasses C₁₋₈alkyl and the higher homologues thereof containing 9 to 12 carbon atoms, such as, for example, nonyl, decyl, undecyl, dodecyl; C₁₋₁₆alkyl again encompasses C₁₋₁₂alkyl and the higher homologues thereof containing 13 to 16 carbon atoms, such as, for example, tridecyl, tetradecyl, pentedecyl and hexadecyl; C₂₋₆alkenyl defines straight and branched chain hydrocarbon radicals containing one double bond and having from 2 to 6 carbon atoms such as, for example, ethenyl, 2-propenyl, 3-butenyl, 2-pentenyl, 3-pentenyl, 3-methyl-2-butenyl, and the like; C₁₋₆alkanediyl defines bivalent straight and branched chained saturated hydrocarbon radicals having from 1 to 6 carbon atoms, such as, for example, methylene, 1,2-ethanediyl, 1,3-propanediyl, 1,4-butanediyl, 1,5-pentanediyl, 1,6-hexanediyl and the branched isomers thereof. The term "C(=O)" refers to a carbonyl group, "S(O)" refers to a sulfoxide and "S(O)₂" to a sulfon.

Unexpectedly, we have now found that the famesyl protein transferase inhibitor identified *supra,* which may hereinafter be referred to as the compound according to the present invention, is useful for the treatment of IBD, including but not limited to Crohn's and ulcerative colitis.

Accordingly, the present invention relates to the use of the famesyl protein transferase inhibitor identified in claim 1 in the manufacture of a medicament for the treatment of inflammatory bowel disease, for example, Crohn's and ulcerative colitis.

The treatment of inflammatory bowel diseases includes both the treatment of the acute disease state, thereby inducing remission of the disease or improvement of the lesions or clinical condition, as well as the use in maintenance therapy.

The compound of the invention is (+)-6-[amino(4-chlorophenyl)(1-methyl-1*H*-imidazol-5-yl)methyl]-4-(3-chlorophenyl)-1-methyl-2(1*H*)-quinolinone (Compound 75 in Table 1 of the Experimental part of WO 97/21701); or a pharmaceutically acceptable acid addition salt thereof.

The pharmaceutically acceptable acid addition salts as mentioned hereinabove are meant to comprise the therapeutically active non-toxic acid addition salt forms which the compound of the invention is able to form. The compound of the invention which has basic properties can be converted in its pharmaceutically acceptable acid addition salts by treating said base form with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic (*i*.*e*. butanedioic acid), maleic, fumaric, malic, tartaric, citric, methanesulfonic, ethanesulfonic, benzenesulfonic, *p*-toluenesulfonic, cyclamic, salicylic, *p*-aminosalicylic, pamoic and the like acids.

The terms acid addition salt also comprise the hydrates and the solvent addition forms which the compound of the invention is able to form. Examples of such forms are e.g. hydrates, alcoholates and the like.

Whenever used hereinafter, the term "compound of the invention is meant to include also the pharmaceutically acceptable acid addition salts.

The compound of the invention can be prepared and formulated into pharmaceutical compositions by methods known in the art and in particular according to the methods described in the published patent specifications mentioned herein and incorporated by reference ; for the compound of the invention suitable examples can be found in WO-97/21701. To prepare the aforementioned medicaments, a therapeutically effective amount of the particular compound, optionally in addition salt form, as the active ingredient is combined in intimate admixture with a pharmaceutically acceptable carrier, which may take a wide variety of forms depending on the form of preparation desired for administration. The carrier(s) must be acceptable in the sense of being compatible with the other ingredients of the formula and not deleterious to the recipient thereof.

These pharmaceutical compositions are desirably in unitary dosage form suitable, preferably, for systemic administration such as oral, rectal, percutaneous, or parenteral administration; or topical administration such as via inhalation, a nose spray, eye drops or via a cream, gel, shampoo or the like. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed.

For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable solutions containing compounds of formula (I) may be formulated in an oil for prolonged action. Appropriate oils for this purpose are, for example, peanut oil, sesame oil, cottonseed oil, com oil, soy bean oil, synthetic glycerol esters of long chain fatty acids and mixtures of these and other oils. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment. As appropriate compositions for topical application there may be cited all compositions usually employed for topically administering drugs e.g. creams, gellies, dressings, shampoos, tinctures, pastes, ointments, salves, powders and the like. Application of said compositions may be by aerosol, e.g. with a propellent such as nitrogen, carbon dioxide, a freon, or without a propellent such as a pump spray, drops, lotions, or a semisolid such as a thickened composition which can be applied by a swab. In particular, semisolid compositions such as salves, creams, gellies, ointments and the like will conveniently be used.

For rectal administration, the pharmaceutical compositions may be presented as suppositories or as enemas. For rectal administration wherein the carrier is a solid, unit dose suppositories are preferred. Suitable carriers include cocoa butter and other materials commonly used in the art.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Preferably, the therapeutic effective amount of the medicament comprising a compound according to the present invention is administered orally or parenterally. Said therapeutically effective amount is the amount that is effective in treating or preventing inflammatory bowel disease. The amount of the compound according to the present invention, which is required to achieve a therapeutic effect will, of course, vary with the route of administration, the age and condition of the recipient, and the particular disorder being treated.

On the basis of the current data, it appears that a pharmaceutical composition comprising (+)-6-[amino(4-chlorophenyl) (1-methyl-1*H*-imidazol-5-yl)methyl]-4-(3-chlorophenyl)-1-methyl-2(1*H*)-quinolinone (compound 75) as the active ingredient can be administered orally in an amount of from 10 to 1500 mg daily, either as a single dose or subdivided into more than one dose. A preferred amount ranges from 100 to 1,000 mg daily, including 50 to 1,000 mg daily. A particularly preferred dosage for such a compound is 300mg administered twice daily. This treatment can be given either continuously or intermittently in cycles of 3-4 weeks with treatment given for 1-21 days per cycle.

The compounds according to the present invention may also be used in accordance with the present invention in combination with one or more IBD treatment agents, including agents known to have utility for alleviating the symptoms of inflammatory bowel disease, including but not limited to:
1. Anti-inflammatory agents, e.g. sulfasalazine, olsalazine
2. Agents that control intestinal tract infection e.g. antibiotics:
   (i) penicillins, e.g. bacitracin
   (ii) glycopeptides, e:g. vancomycin
3. Steroids corticosteroids , e.g. prednisolone budenoside
4. Immuno-modulating agents, e.g. azathioprine, 6-MP
5. Agents for controlling diarrhea, e.g; cholestryramine

The various components of the combination described herein may be administered simultaneously (e.g. in separate or unitary compositions) or sequentually in either order and optionally via different routes.

### Testing Method for Inflammatory Bowel Disease

The purpose of this study was to identify, characterize and document the preventative actions of compound 75 above, namely (+)-6-[amino(4-chlorophenyl) (1-methyl-1*H*-imidazol-5-yl)methyl]-4-(3-chlorophenyl)-1-methyl-2(1*H*)-quinolinone, a compound of formulae (I), in an experimental model of inflammatory bowel disease in mice.

### Procedure A group of female Swiss-Webster mice was received from Ace Animals, Inc., Boyertown, PA. The animals were grouped housed (5 mice/cage) in plastic cages with water absorbent bedding. The animal room was temperature controlled and had a 12-hour light/dark cycle. The animals were fed Purina Rodent Chow #5012 and filtered tap water was supplied ad libitum by automatic watering system (during acclimation).

Following an acclimation period of 9 days, 40 healthy mice were selected for this study. The test animals were distributed (10 mice/group) into each one of the 4 test groups described below. There were no statistical significant differences between the mean group body weights.

| **GROUP NUMBER** | **PRODUCT ID** | **DOSE LEVEL (mg/kg)** |
|---|---|---|
| 1 | Vehicle no. DSS | 0 |
| 2 | Vehicle with DSS | 0 |
| 3 | Compound 75 with DSS | 25 PO bid |
| 4 | Compound 75 with DSS | 50 PO bid |

| | | |
|---|---|---|
| PO - oral bid- twice per day (4 to 6 hours apart) days 0 through 6 | | |

Individual doses were calculated based on daily body weights. Prior to administration, Compound 75 was mixed with the vehicle (Beta cyclodextrin (40 %) in 0.1 N HCL). This mixture was further diluted with 0.1 N HCL. The resulting mixture was then used to prepare the dilutions for groups 3 and 4. A 20 % solution of Beta cyclodextrin in 0.1 N HCL was used for Groups 1 and 2 and constituted the vehicle control. Groups 2 through 4 were given a 5 % w/w solution of dextran sulfate sodium (DSS) in distilled water substituted for their normal water supply (approximately 10 ml/mouse/day) from Days 0 through 6. Each group of animals received the appropriate amount of the test substance, as described above, by intubation using a stainless steel ball-tipped gavage needle attached to an appropriate syringe.

All mice were weighed daily and observed for signs of gross toxicity and behavioural changes, consistency of stool and presence of gross blood during the study. On Day 7, all animals were euthanized by CO₂ inhalation and necropsied. Following euthanasia, a stool sample was obtained from the colon of each animal and tested for occult blood (Quik-Cult, Laboratory Diagnostics Co., Morganville, NJ). The colons were then removed and the length from the colo-cecal junction to the end of the distal rectum was measured. A segment of colon was collected from each animal and preserved in 10 % formalin.

For each group, the disease activity index (DAI) was determined by evaluating changes in weight, Hemoccult positivity or gross bleeding and stool consistency using the following system.

### Criteria for Scoring Disease Activity Index*

| Score | Weight Loss (%) | Stool # | Blood in feces |
|---|---|---|---|
| 0 | 0 or gain | Normal | Negative |
| 1 | 1-4.9 | ---- | +/- |
| 2 | 5.0-9.9 | Semi-solid | + |
| 3 | 10.15 | Diarrhoea | ++ |
| 4 | >15 | Bloody diarrhoea | Gross blood |

| | | | |
|---|---|---|---|
| * DAI = (combined score of weight loss, stool consistency and bleeding)/3. | | | |
| # Normal stools = well-formed pellets; semi-solid = pasty stool that does not stick to the anus; diarrhoea = liquid stools that stick to the anus. | | | |

Data was analyzed using analysis of variance (single factor). Statistically methods included the Mann-Whitney test, ANOVA and Turkey-Kramer Multiple Comparisons test.
Statistical significance between test and control groups was established at a probability of p<0.05.

### Results and conclusion

Compound 75 showed significant activity in prevention of colitis produced by 5 % Dextran Sulfate Sodium. Dose levels of 25 and 50 mg/kg PO bid prevented the shortening of the colon produced by DSS. The lower dose may have been more effective than the 50 mg/kg dose. Similar results were observed with the DAI (disease activity index) with or without the weight parameter. No significant protection against weight loss was noted with any dose. However, control mice receiving cyclodextrin lost weight over the duration of the study. In most studies vehicle treated mice usually gain weight. The weight loss observed with cyclodextrin + DSS was greater than that normally observed. Due to the severity of the weight loss in the control DSS group the study was terminated one day early. Eight deaths were noted in the study, 2 in the vehicle + DSS group, 1 each at the 25 and 50 mg/kg dose.

**TABLE 1: EFFECT OF Compound 75 AFTER PO ADMINISTRATION BID: PREVENTION OF DSS-INDUCED COUTIS IN MICE**

| Treatment (mg/kg) + DSS | Mean Colon Length Cm±SEM | % of Normal Length Shortening | DAI Mean ± SEM | DAINWT Mean ± SEM | Blood in Feces (%) |
|---|---|---|---|---|---|
| 1 Veh. PO no DSS | 11.8±0.1 | 100 | 0.43±0.11 | 0.20±0.13 | 0 |
| 2 Veh. PO | 8.4±0.2 | 70.5 | 2.96±0.34 | 3.06±0.29 | 50 |
| 3 Compound 75 25 mg/kg PO | 9.7±0.3# | 82.0 | 1.70±0.26* | 1.50±0.22 | 0 |
| 4 Compound 75 50 mg/kg PO | 9.2±0.1# | 77.9 | 2.11 1±0.25* | 2.06±0.28 | 22 |

| | | | | | |
|---|---|---|---|---|---|
| PO - oral | | | | | |
| DAI - Disease activity index | | | | | |
| DAINWT - Disease activity index without the body weight parameter | | | | | |
| * Statistically significant difference from Vehicle + DSS group p<0.05 Mann-Whitney test | | | | | |
| # Significant difference from Vehicle + DSS group p<0.05 one way ANOVA and Turkey-Kramer Multiple Comparisons test | | | | | |

**TABLE 2: EFFECT OF Compound 75 IN PERCENT WEIGHT CHANGE FROM DSS-INDUCED COLITIS IN MICE**

| Treatment + DSS Mg/kg | Mean Weight (grams) and % weight change (± SEM) | | | | | | |
|---|---|---|---|---|---|---|---|
| | DAY 0 | DAY 5 | % change | DAY 6 | % change | DAY 7 | % change |
| 1 Veh. PO no DSS | 25.2±0.4 | 23.9±0.4 | -5.2±1.0 | 24.4±0.5 | -3.2±1.1 | 24.3±0.5 | -3.6±1.1 |
| 2 Veh. PO | 24.9±0.4 | 23.4±0.5 | -5.9±2.2 | 23.5±0.9 | -5.5±3.6 | 21.9±0.6 | -12.0±2.6 |
| 3 Compound 75 25 mg/kg PO | 25.1±0.5 | 23.0±0.5 | -8.3±1.5 | 22.9±0.7 | -8.7±2.7 | 22.7±0.9 | -9.6±3.5 |
| 4 Compound 75 50 mg/kg PO | 25.6±0.5 | 23.1±0.5 | -9.5±1.3 | 23.3±0.8 | -8.8±2.2 | 23.2±0.7 | -9.2±1.9 |

| | | | | | | | |
|---|---|---|---|---|---|---|---|
| PO - oral | | | | | | | |

In view of the above results the experiment was repeated with lower doses of compound 75. The above procedure was followed with the following groups

| GROUP NUMBER | PRODUCT ID | DOSE LEVEL (mg/kg) |
|---|---|---|
| 1 | Vehicle no DSS | 0 |
| 2 | Compound 75 with DSS | 0 |
| 3 | Compound 75 with DSS | 12.5 PO |
| 4 | Compound 75 with DSS | 25 PO |
| 5 | Compound 75 with DSS | 50 PO |

| | | |
|---|---|---|
| PO - oral | | |

Individual doses were calculated based on daily body weights. Prior to administration, 50 mg of Compound 75 was diluted with 10 milliliters of 0.1 N HCI (50 mg/kg for Group 5). The resulting solution was then further diluted twice, with 5 ml increments and used for the 25 (Group 4) and 12.5 (Group 3) mg/kg dose levels. The 0.1 N HCI was used for Groups 1 and 2 and constituted the vehicle control. Groups 2 through 5 were given a 5% w/w solution of dextran sulfate sodium (DSS) in distilled water substituted for their normal water supply (approximately 10 ml:mouse/day) from Days 0 through 7. Each group of animals received the appropriate amount of the test substance, as described above, by intubation using a stainless steel ball-tipped gavage needle attached to an appropriate syringe.

### Results and conclusion

In this study, DSS produced a typical degree of colonic inflammation and colonic shortening. However, there was not the significant loss of body weight observed in other studies. The use of 0.1 N HCI may have been a factor in the lack of weight loss. The use of the DAI, in which weight loss is an important component, underestimates the degree of colitis and therefore the disease activity index using presence of fecal blood and stool consistency (DAINWT) is probably a more reliable estimate of activity. The typical degree of colonic shortening was observed and this measure remains the most significant indication of the extent of colitis and the degree protection. Compound 75 produced a statistically significant protection at all doses based on DAI and DAINWT. The maximal effectiveness on prevention of colonic shortening was observed at the lowest dose tested (12.5 mg/kg PO daily). The degree of effectiveness on this compound compares favorably with other experimental compounds, which have been tested previously and also confirms the initial test done with twice a day dosing and administered in cyclodextrin. No deaths were observed during the duration of the experiment.

### TABLE 1: EFFECT OF COMPOUND 75 AFTER PO ADMINISTRATION: PREVENTION OF DSS-INDUCED COLITIS IN MICE

| Treatment (mg/kg) + DSS | Mean Colon Length Cm ± SEM | % of Normal Length Shortening | DAI mean ± SEM | DAINWT mean ± SEM | % Inh. Colonic Shortening |
|---|---|---|---|---|---|
| 1. Veh. PO no DSS | 11.2±0.2 | 100 | 0.11±0.06* | 0.06±0.06* | - |
| 2. Veh. PO | 8.2±0.2 | 73.1 | 2.26±0.13 | 3.17±0.12 | 0 |
| 3. Compound 75 12.5 mg/kg PO | 10.0±0.1# | 89.6 | 1.57±0.17* | 2.25±0.25* | 65 |
| 4. Compound 75 25 mg/kg PO | 9.9±0.3# | 88.7 | 1.53±0.17* | 2.30±0.25* | 58 |
| 5. Compound 75 50 mg/kg PO | 9.7±0.2# | 87.0 | 1.70±0.18* | 2.30±0.24* | 52 |

| | | | | | |
|---|---|---|---|---|---|
| PO- oral | | | | | |
| DAI- Disease Activity Index | | | | | |
| DAINW- Disease Activity Index without the body weight parameter | | | | | |
| * Statistically significant difference from Vehicle + DSS group p< 0.05 Mann-Whitney Test | | | | | |
| # Significant difference from Vehicle + DSS group p< 0.05 one way ANOVA and Turkey-Kramer Multiple Comparisons Test | | | | | |

**Table 2: EFFECT OF COMPOUND 75 IN PERCENT WEIGHT CHANGE FROM DSS- INDUCED COLITIS IN MICE**

| *Treatment (mg*/*kg + DSS* | *DAY 0* | *DAY 5* | *% change* | *DAY 6* | *% change* | *DAY 7* | *% Change* |
|---|---|---|---|---|---|---|---|
| 1.Veh. PO no DSS | 23.8±0.4 | 23.7±0.6 | -0.5±2.0 | 23.1±0.5 | -2.8±1.0 | 24.0±0.5 | +0.9±1.0 |
| 2.Veh. PO | 24.6±0.2 | 24.2±0.4 | -1.3±1.0 | 24.1±0.3 | -1.8±1.0 | 24.6±0.4 | 0.0±2.0 |
| 3.Compound 75 12.5 mg/kg PO | 23.6±0.3 | 23.9±0.4 | +1.3±1.0 | 23.6±0.4 | 0.0±1.0 | 23.9±0.4 | +1.3±1.0 |
| 4.Compound 75 25 mg/kg PO | 24.2±0.4 | 24.7±0.6 | +2.0±1.0 | 23.9±0.5 | -1.2±1.0 | 24.9±0.5 | +2.9±1.0 |
| 5.Compound 75 50 mg/kg PO | 23.9±0.3 | 23.9±0.3 | -0.7±1.0 | 23.5±0.3 | -1.6±1.0 | 23.8±0.3 | -0.3±2.0 |

| | | | | | | | |
|---|---|---|---|---|---|---|---|
| PO - Oral | | | | | | | |

### Diagnostic Criteria for Inflammatory bowel Disease

The diagnostic criteria for inflammatory bowel disease are those found in standard medical references e.g., Harrison's Principles of Internal Medicine, thirteenth ed., 1994, by McGraw-Hill, Inc., ISBN 0-07-032370-4, pgs. 1194-1197. These criteria may be used to determine when to begin using the method of the invention, the frequency and degree of treatment, and the time for cessation of treatment.

While the present invention has been illustrated above by certain specific embodiments, these are not intended to limit the scope of the invention as described in the appended claims.

## Claims

1. Use of (+)-6-[amino(4-chlorophenyl)(1-methyl-1*H*-imidazol-5-yl)methyl]-4-(3-chloro-phenyl)-1-methyl-2(1*H*)-quinolinone or a pharmaceutically acceptable acid addition salt thereof in the manufacture of a medicament for the treatment of inflammatory bowel disease.

2. Use according to claim 1 for the treatment of Crohn's disease.

3. Use according to claim 1 for the treatment of ulcerative colitis.

4. Use of any of claims 1 to 3 wherein the medicament is adapted for oral, rectal or parental administration.

5. A pharmaceutical combination comprising a compound as defined in claim 1 and one or more inflammatory bowel disease treatment agents.

## Revendications

1. Utilisation de (+)-6-[amino(4-chlorophényl)(1-méthyl-1*H*-imidazol-5-yl)méthyl]-4-(3-chlorophényl)-1-méthyl-2(1*H*)quinolinone ou d'un sel d'addition d'acide pharmaceutiquement acceptable de celle-ci dans la fabrication d'un médicament destiné au traitement de l'affection abdominale inflammatoire.

2. Utilisation selon la revendication 1, destinée au traitement de la maladie de Crohn.

3. Utilisation selon la revendication 1, destinée au traitement de la rectocolite ulcérative.

4. Utilisation selon l'une quelconque des revendications 1 à 3, dans laquelle le médicament est adapté pour une utilisation par voie orale, rectale ou parentérale.

5. Association pharmaceutique, comprenant un composé tel que défini selon la revendication 1 et un ou plusieurs agents de traitement de l'affection abdominale inflammatoire.

## Patentansprüche

1. Verwendung von (+)-6-[Amino(4-chlorphenyl)(1-methyl-1-*H*-imidazol-5-yl)methyl]-4-(3-chlorphenyl) -1-methyl-2(1*H*)-chinolinon oder eines pharmazeutisch unbedenklichen Säureadditionssalzes davon bei der Herstellung eines Medikaments zur Behandlung von entzündlicher Darmerkrankung.

2. Verwendung nach Anspruch 1 zur Behandlung von Morbus Crohn.

3. Verwendung nach Anspruch 1 zur Behandlung von Colitis ulcerosa.

4. Verwendung nach einem der Ansprüche 1 bis 3, wobei das Medikament für die orale, rektale oder parenterale Verabreichung ausgelegt ist.

5. Pharmazeutische Kombination, enthaltend eine wie in Anspruch 1 definierte Verbindung und ein oder mehrere Mittel zur Behandlung von entzündlicher Darmerkrankung.
